# EUROPÄISCHE PATENTSCHRIFT

(11) **EP 0 899 564 B1**
(45) Veröffentlichungstag und Bekanntmachung des Hinweises auf die Patenterteilung: **25.08.2010**
(21) Anmeldenummer: 98116031.0
(22) Anmeldetag: 25.08.1998
(51) Int. Cl.: G01N 29/02, A61M 5/36, G01N 29/12

(54) **Ultraschallsender, insbesondere für einen Luftblasendetektor**
Ultrasonic transmitter, in particular for air-bubble detection
Emetteur ultrasonique, en particulier pour la détection de bulles d'air

(30) Priorität: 01.09.1997 DE 19738146
(43) Veröffentlichungstag der Anmeldung: 03.03.1999
(73) Patentinhaber: Fresenius AG, 61352 Bad Homburg (DE)
(72) Erfinder: Meisberger, Artur, 66606 St. Wendel (DE)
(74) Vertreter: Gossel, Hans K.

(56) Entgegenhaltungen:
- EP-A- 0 496 436
- EP-A- 0 643 301
- US-A- 4 015 464
- US-A- 4 736 192

## Beschreibung

Die Erfindung betrifft einen Ultraschallsender, insbesondere für einen Luftblasendetektor, mit einer Sendestufe und mit einem Ultraschallschwinger. Der Ultraschallschwinger besteht üblicherweise aus einem Piezo-Element, das auf einer seiner Resonanzfrequenzen betrieben wird. Hierbei stellt sich das Problem, eine Sendestufe bereitzustellen, die den Ultraschallschwinger auf eben dieser Resonanzfrequenz anregt, damit am Ultraschallschwinger ein möglichst starkes Ausgangssignal erzeugt wird.

Wird der Ultraschallsender in einem Luftblasendetektor eingesetzt, so sind an den Ultraschallsender auch besondere Anforderungen hinsichtlich der Zuverlässigkeit zu stellen. Bei einem Luftblasendetektor befindet sich zwischen dem Ultraschallsender und einem entsprechend angeordneten Ultraschallempfänger ein Schlauch, der auf Luftblasen überwacht werden soll. Bei der Verabreichung von Infusionen oder bei der Durchführung von Transfusionen muß ein Lufteintritt in die hierbei verwendeten Schläuche unbedingt erkannt werden, da für den Patienten ansonsten lebensbedrohliche Situationen entstehen können. Zur Erkennung der Luftblasen wird dabei die Tatsache ausgenutzt, daß sich die Dämpfung der Ultraschallstrecke verändert, sobald eine Luftblase in den zwischen dem Ultraschallsender und dem Ultraschallempfänger befindlichen Schlauch eintritt.

Aus der US 5 583 280 ist ein Luftblasendetektor mit einer Sendestufe bekannt, die einen Frequenzgenerator umfaßt, der innerhalb eines bestimmten Frequenzbereiches linear durchgestimmt wird, wobei eine Resonanzfrequenz des Ultraschallschwingers ebenfalls in diesem Frequenzbereich liegt. Sobald der durchstimmende Frequenzgenerator die Resonanzfrequenz des Ultraschallschwingers trifft, findet eine Anregung des Ultraschallschwingers statt und die Durchstimmung des Frequenzgenerators kann von neuem beginnen.

Aus der EP 0 416 911 A2 ist ebenfalls ein Luftblasendetektor mit einer Sendestufe bekannt, die einen variablen Frequenzgenerator aufweist, wobei zusätzlich eine Testschaltung zum Erkennen von Fehlfunktionen vorgesehen ist.

Aus der EP 0 340 470 A1 ist ein Flüssigkeitszerstäuber mit einem Ultraschallschwinger bekannt, wobei zur Anregung des Ultraschallschwingers ein spannungsgesteuerter Oszillator vorgesehen ist. Dieser wird mit einem Dreieckgenerator so geregelt, daß seine Frequenz in einem die Serienresonanz des Ultraschallschwingers umschließenden Bereich periodisch gewobbelt wird. Mit Hilfe einer überlagerten Regelschleife läßt sich erreichen, daß der spannungsgesteuerte Oszillator auf der Serien-Resonanzfrequenz des Ultraschallschwingers einrastet.

Aus der EP 0 084 485 A2 ist ebenfalls ein Flüssigkeitszerstäuber mit einem Ultraschallschwinger bekannt. Zur Anregung des Ultraschallschwingers sind ein Multivibrator und ein Impuls-Generator derart verschaltet, daß der Impulsgenerator im Takt der Eigenfrequenz des Multivibrators Impulse an den Ultraschallschwinger abgibt. Der Impuls wirkt auf den Ultraschallschwinger als eine Systemanregung, so daß der Ultraschallschwinger auf den Impuls mit einer gedämpften Resonanzschwingung reagiert. Beim Impulsbetrieb besteht allerdings grundsätzlich der Nachteil, daß immer ein Impulsgenerator mit einem entsprechenden Energiespeicher zur Bereitstellung der Impulsenergie benötigt wird, was einen verhältnismäßig hohen Schaltungsaufwand zur Folge hat.

Weiterhin ist aus der US 5,583,280 ein Flüssigkeitsstandanzeiger nach dem Ultraschallprinzip bekannt, wobei zur Anregung des Ultraschallschwingers ein rückgekoppeltes Bandpaßfilter verwendet wird, das ähnlich wie bei einer PLL-Schaltung auf die Resonanzfrequenz des Ultraschallschwingers einrastet.

Ein Nachteil der aus dem Stand der Technik bekannten Ultraschallsender besteht darin, daß die Sendestufen einen verhältnismäßig hohen Schaltungsaufwand aufweisen.

Aufgabe der Erfindung ist es daher, einen Ultraschallsender zu schaffen, dessen Sendestufe zur Anregung des sendeseitigen Ultraschallschwingers einfach aufgebaut ist und gleichzeitig ein starkes Ausgangssignal am Ultraschallschwinger erzeugt.

Diese Aufgabe wird mit den Merkmalen des Patentanspruchs 1 gelöst.

Die erfindungsgemäße Lösung besteht darin, daß die Sendestufe einen Multivibrator aufweist, der in an sich bekannter Weise aus einer Kippschaltung und einem im Rückkopplungszweig der Kippschaltung verschalteten Zeitglied besteht. In dieser Form schwingt der Multivibrator bereits selbsttätig auf seiner Eigenfrequenz an, die im wesentlichen durch das Zeitglied beeinflußt ist. Erfindungsgemäß wird der Ultraschallschwinger mit dem Rückkopplungszweig der Kippschaltung derart verschaltet, daß die Sendestufe auf oder in der Nähe einer Resonanzfrequenz des Ultraschallschwingers schwingt. Im Gegensatz zu bekannten Anregerschaltungen arbeitet der Ultraschallschwinger damit selbst als frequenzbestimmendes Bauteil für eine kontinuierlich erzeugte Sendefrequenz. Dies spart erheblichen Schaltungsaufwand, gleichzeitig erhält man dadurch eine besonders unempfindliche Anordnung gegenüber elektromagnetischen Störungen, wodurch wiederum die Zuverlässigkeit erhöht wird. Der erfindungsgemäße Ultraschallsender generiert zudem ein sehr starkes Ausgangssignal, das wiederum eine einfach aufzubauende Empfängerschaltung ermöglicht.

Nach einer bevorzugten Ausführungsform ist vorgesehen, daß das Zeitglied des Multivibrators aus mindestens einem RC-Glied besteht und daß der Ultraschallschwinger parallel zum Widerstand eines RC-Gliedes geschaltet ist. Auf diese Weise ist ein sicheres Anschwingen der Schaltung gewährleistet, da der Multivibrator zunächst sicher auf seiner Eigenfrequenz anschwingt und sodann aufgrund der sprunghaften Pegeländerungen am Ausgang der Kippschaltung den Ultraschallschwinger auf einer Resonanzfrequenz anregt.

Nach einer weiteren bevorzugten Ausführungsform ist am Ausgang der Kippschaltung ein Tiefpaß vorgesehen, um ein Anschwingen des Ultraschallschwingers auf dessen Oberschwingungen zu unterdrücken. Ein definiertes Anschwingen kann zusätzlich auch dadurch erreicht werden, daß die Sendestufe unterhalb der entsprechenden Resonanzfrequenz des Ultraschallschwingers schwingt.

Zweckmäßigerweise wird der Ultraschallschwinger auf seiner untersten Serien-Resonanzfrequenz betrieben. Handelt es sich bei dem Ultraschallschwinger beispielsweise um eine Piezo-Element, so ist die Serien-Resonanzfrequenz von äußeren Einflüssen weitgehend unabhängig, während in die Parallelresonanzfrequenz die schlecht definierte Elektrodenkapazität des Piezo-Elements eingeht.

Nach einer weiteren bevorzugten Ausführungsform ist vorgesehen, daß die Sendestufe logisch mit einem Testeingang verschaltet ist, um den Ultraschallsender zu Testzwecken gezielt zu aktivieren bzw. zu deaktivieren. Zweckmäßigerweise ist dabei der Testeingang mit einem Und-Gatter mit dem Eingang der Kippschaltung verschaltet. Auf diese Weise kann der Ultraschallsender für sicherheitstechnische Anwendungen gezielt getestet werden.

Nach einer weiteren bevorzugten Ausführungsform ist vorgesehen, daß die Kippschaltung ein Schmitt-Trigger ist. Ein herkömmlicher Schmitt-Trigger ist als integriertes Bauelement leicht verfügbar und wird durch ein Zeitglied im Rückkopplungszweig kaum belastet, so daß sich für die Wahl der Bauelemente des Zeitgliedes ein großer Spielraum ergibt.

Ein Luftblasendetektor, für den selbständiger Schutz beansprucht wird, besteht aus dem erfindungsgemäßen Ultraschallsender, aus einem Ultraschallempfänger und einem zwischen Ultraschallsender und Ultraschallempfänger eingebrachten Schlauch.

Weitere Einzelheiten und Vorteile der Erfindung werden anhand mehrerer in der Zeichnung dargestellter Ausführungsbeispiele erläutert. In dieser zeigt:
- Fig. 1: eine elektrische Schaltung eines Luftblasendetektors mit dem erfindungsgemäßen Ultraschallsender,
- Fig. 2: einen bekannten Multivibrator,
- Fig. 3: die Verschaltung eines Ultraschallschwingers mit dem Rückkopplungszweig des Multivibrators gemäß Fig. 2 und
- Fig. 4: den schematischen Aufbau des Luftblasendetektors.

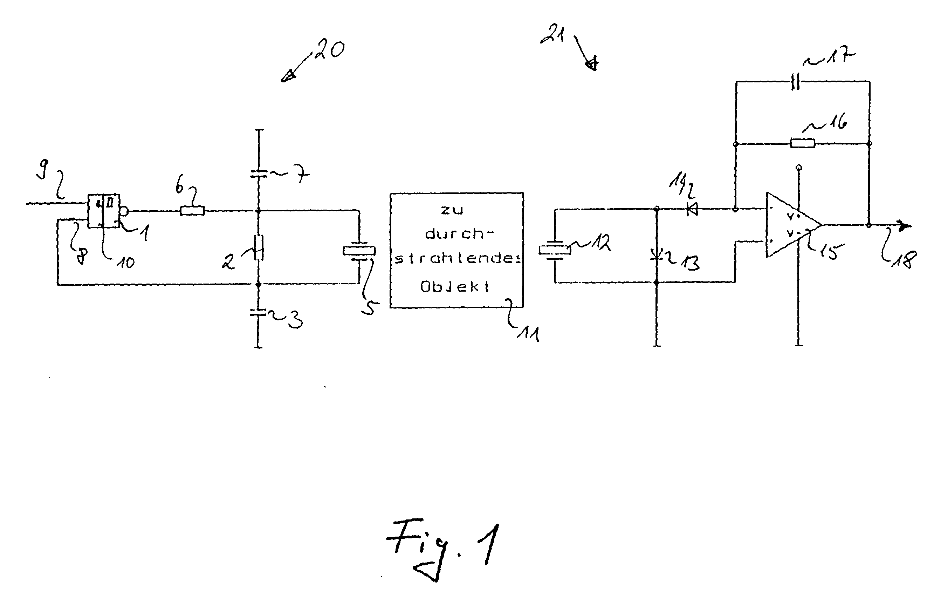

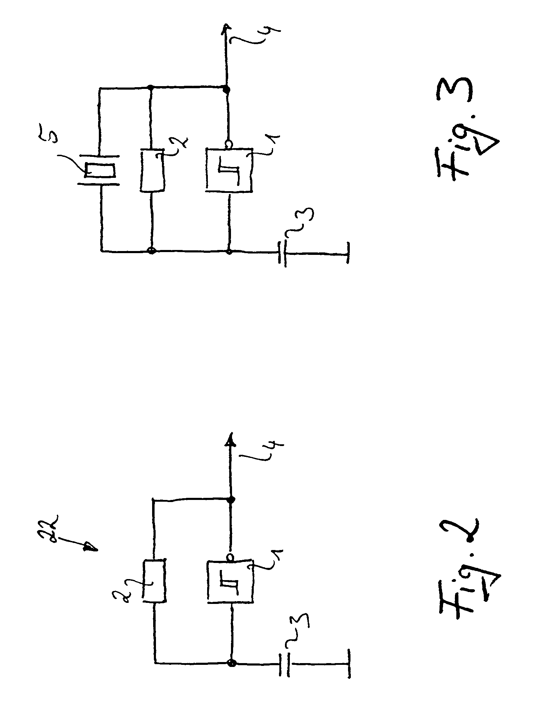

Bevor auf die Funktion des Ultraschallsenders gemäß Fig. 1 eingegangen wird, wird das Funktionsprinzip anhand der Fig. 2 und 3 erläutert. Fig. 2 zeigt zunächst den Aufbau eines bekannten Multivibrators. Der Multivibrator 22 weist als Kippschaltung einen Schmitt-Trigger 1 auf, der in seinem Rückkopplungszweig mit dem Widerstand 2 und dem Kondensator 3 verschaltet ist. Der Multivibrator schwingt selbsttätig auf seiner Eigenfrequenz, wobei der Kondensator 3 über den Widerstand 2 bis zum Ausschaltpegel des Schmitt-Triggers aufgeladen und anschließend wieder bis zum Einschaltpegel entladen wird. Der Schmitt-Trigger 1 weist an seinem Ausgang zwei stabile Zustände auf, so daß am Ausgang 4 ein periodisches Rechtecksignal entsteht.

Fig. 3 zeigt die Verschaltung eines Ultraschallschwingers mit dem Rückkopplungszweig des Multivibrators gemäß Fig. 2. Der Ultraschallschwinger besteht dabei aus einem Piezo-Element 5, wobei das Piezo-Element 5 parallel zu dem Widerstand 2 geschaltet ist. Nach dem Einschalten wird die Schaltung zunächst auf der Eigenfrequenz des Multivibrators anschwingen. Aufgrund der Rechtecksignale am Ausgang wird allerdings das Piezo-Element 5 ebenfalls durch Rechtecksignale angeregt, wodurch am Eingang des Schmitt-Triggers 1 ein periodisches Signal mit der Resonanzfrequenz des Piezo-Elements 5 entsteht. Hierdurch wiederum werden auch am Ausgang 4 Rechtecksignale mit der Frequenz der Resonanzfrequenz des Piezo-Elements 5 erzeugt, so daß die Schaltung schließlich auf der Resonanzfrequenz des Piezo-Elements schwingt.

Fig. 1 zeigt eine elektrische Schaltung eines Luftblasendetektors mit dem erfindungsgemäßen Ultraschallsender. Der Luftblasendetektor besteht aus einem Ultraschallsender 20, einem Ultraschallempfänger 21 und einem dazwischen eingebrachten medizinischen Schlauch 11, bei dem Luftblasen sicher detektiert werden sollen. Der Ultraschallsender 20 unterscheidet sich gegenüber der Schaltung gemäß Fig. 3 dadurch, daß am Ausgang des Schmitt-Triggers 1 zusätzlich ein Tiefpaß 6, 7 vorgesehen ist und daß der Eingang des Schmitt-Triggers mit einem Testeingang 9 logisch verschaltet ist. Der Tiefpaß besteht aus dem Widerstand 6 und dem Kondensator 7 und bewirkt, daß der Ultraschallschwinger zum einen sicher auf seiner untersten Resonanzfrequenz anschwingt und daß zum anderen die Spannung am Ultraschallschwinger 5 annähernd sinusförmig ist. Die Verschaltung des Testeingangs 9 mit dem Rückkopplungszweig 8 erfolgt durch ein Und-Gatter 10. Hierdurch kann der Schmitt-Trigger 1 zu Testzwecken sicher aktiviert oder deaktiviert werden. Auf der gegenüberliegenden Seite des medizinischen Schlauches 11 befindet sich ein Ultraschallempfänger 21, der ein baugleiches Piezo-Element 12 aufweist. Die Spannung am Piezo-Element 12 wird durch die Dioden 13, 14 gleichgerichtet und über den Operationsverstärker 15 über den Widerstand 16 und den Kondensator 17 geglättet, so daß am Ausgang 18 das entsprechende Hüllkurvensignal anliegt. Gelangt eine Luftblase in den medizinischen Schlauch 11, so verändert sich gegenüber dem mit Flüssigkeit gefüllten Schlauch die Dämpfung in der Ultraschallstrecke zwischen Ultraschallsender 20 und Ultraschallempfänger 21. Hierdurch wiederum verändert sich das Hüllkurvensignal am Ausgang 18, wodurch eine Detektion von Luftblasen innerhalb des Schlauches 11 möglich ist.

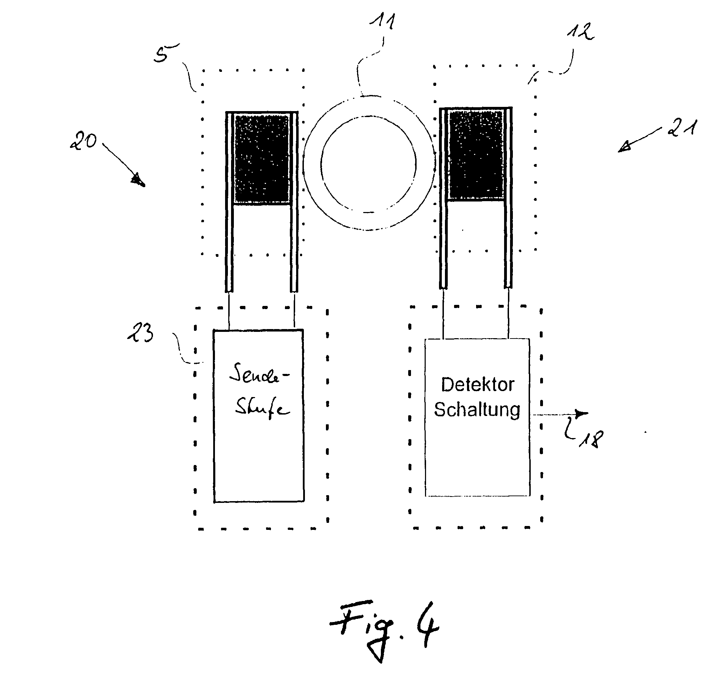

Fig. 4 zeigt den schematischen Aufbau des Luftblasendetektors gemäß Fig. 1 mit einem Ultraschallsender 20 und einem Ultraschallempfänger 21. Die Ultraschallschwinger 5, 12 bestehen im wesentlichen aus einer Piezoscheibe mit zwei Anschlüssen. Diese sind an dem zu überwachenden Schlauch 11 gegenüberliegend angeordnet, so daß der Schall vom sendeseitigen Ultraschallschwinger 5 zum empfangsseitigen Ultraschallschwinger 12 gelangen kann. Wegen der nötigen Anpassung an die akustische Impedanz des medizinischen Schlauches sind die Piezoscheiben mit einem für diesen Zweck geeigneten Koppelmedium an den medizinischen Schlauch angekoppelt. Aus diesem Grund und zum Schutz vor Beschädigung sind die Ultraschallschwinger in ein Gehäuse eingegossen, das gleichzeitig das entsprechende Lager für den zu überwachenden Schlauch bildet. Der sendeseitige Ultraschallschwinger 5 wird von der Sendestufe 23 derart angesteuert, daß der Ultraschallschwinger 5 auf seiner untersten Resonanzfrequenz schwingt. Der empfangsseitige Ultraschallschwinger 12 ist gleich aufgebaut wie der sendeseitige Ultraschallschwinger, so daß dessen größte Empfindlichkeit auf die Sendefrequenz des Ultraschallsenders 20 fällt. Auf diese Weise erhält man ein größtmögliches Ausgangssignal 18 am Ultraschallempfänger 21, das einfach auszuwerten ist.

## Patentansprüche

1. Ultraschallsender (20), insbesondere für einen Luftblasendetektor, mit einer Sendestufe (23) und mit einem Ultraschallschwinger (5),
**dadurch gekennzeichnet,**
**daß** die Sendestufe (23) einen Multivibrator (22) aufweist, der aus einer zwei stabile Ausgangszustände aufweisenden Kippschaltung (1) und einem im Rückkopplungszweig der Kippschaltung verschalteten Zeitglied (2, 3) besteht, und
**daß** der Ultraschallschwinger (5) mit dem Rückkopplungszweig der Kippschaltung derart verschaltet ist, daß die Sendestufe (23) auf oder in der Nähe einer Resonanzfrequenz des Ultraschallschwingers schwingt.

2. Ultraschallsender nach Anspruch 1, **dadurch gekennzeichnet, daß** das Zeitglied des Multivibrators aus mindestens aus einem RC-Glied (2, 3) besteht.

3. Ultraschallsender nach Anspruch 2, **dadurch gekennzeichnet, daß** der Ultraschallschwinger (5) parallel zum Widerstand (2) des RC-Gliedes geschaltet ist.

4. Ultraschallsender nach einem der Ansprüche 1 bis 3, **dadurch gekennzeichnet, daß** am Ausgang der Kippschaltung ein Tiefpaß (6, 7) vorgesehen ist, um ein Anschwingen des Ultraschallschwingers (5) auf dessen Oberschwingungen zu unterdrücken.

5. Ultraschallsender nach Anspruch 4, **dadurch gekennzeichnet, daß** der Ultraschallschwinger (5) auf seiner untersten Serien-Resonanzfrequenz betrieben wird.

6. Ultraschallsender nach einem der Ansprüche 1 bis 5, **dadurch gekennzeichnet, daß** die Sendestufe unterhalb einer entsprechenden Resonanzfrequenz des Ultraschallschwingers schwingt.

7. Ultraschallsender nach einem der Ansprüche 1 bis 6, **dadurch gekennzeichnet, daß** die Sendestufe (23) logisch mit einem Testeingang (9) verschaltet ist, um den Ultraschallsender zu Testzwecken gezielt zu aktivieren bzw. zu deaktivieren.

8. Ultraschallsender nach Anspruch 7, **dadurch gekennzeichnet, daß** der Testeingang (9) mit einem Und-Gatter (10) mit dem Eingang der Kippschaltung (1) verschaltet ist.

9. Ultraschallsender nach einem der Ansprüche 1 bis 8, **dadurch gekennzeichnet, daß** der Ultraschallschwinger (5) ein Piezo-Element ist.

10. Ultraschallsender nach einem der Ansprüche 1 bis 9, **dadurch gekennzeichnet, daß** die Kippschaltung (1) ein Schmitt-Trigger ist.

11. Luftblasendetektor mit einem Ultraschallsender (20) nach einem der Ansprüche 1-10, mit einem Ultraschallempfänger (21) und mit einem zwischen Ultraschallsender (20) und Ultraschallempfänger (21) eingebrachten Schlauch (11).

## Claims

1. An ultrasonic transmitter (20), in particular for an air bubble detector, having a transmitting stage (23) and having an ultrasound vibrator (5),
**characterized in that**
the transmitting stage (23) has a multivibrator (22) which comprises a flip-flop circuit (1) having two stable output states and a timing element (2, 3) connected in the feedback loop of the flip-flop circuit; and
**in that** the ultrasonic vibrator (5) is connected to the feedback loop of the flip-flop circuit such that the transmitting stage (23) oscillates at or near a resonance frequency of the ultrasonic vibrator.

2. An ultrasonic transmitter in accordance with claim 1, **characterised in that** the timing element of the multivibrator comprises at least one RC element (2, 3).

3. An ultrasonic transmitter in accordance with claim 2, **characterised in that** the ultrasonic vibrator (5) is connected in parallel to the resistor (2) of the RC element.

4. An ultrasonic transmitter in accordance with one of claims 1 to 3, **characterised in that** a low-pass filter (6, 8) is provided at the output of the flip-flop circuit to suppress an oscillation build-up of the ultrasonic vibrator (5) to its harmonics.

5. An ultrasonic transmitter in accordance with claim 4, **characterised in that** the ultrasonic vibrator (5) is operated at its lowest series resonant frequency.

6. An ultrasonic transmitter in accordance with one of the claims 1 to 5, **characterised in that** the transmitting stage oscillates below a corresponding resonant frequency of the ultrasonic vibrator

7. An ultrasonic transmitter in accordance with one of claims 1 to 6, **characterised in that** the transmitting stage (23) is logically connected to a test input (9) to activate or deactivate the ultrasonic transmitter for test purposes.

8. An ultrasonic transmitter in accordance with claim 7, **characterised in that** the test input (9) having an And gate (10) is connected to the input of the flip-flop circuit (1).

9. An ultrasonic transmitter in accordance with one of the claims 1 to 8, **characterised in that** the ultrasonic vibrator (5) is a piezoelectric element.

10. An ultrasonic transmitter in accordance with one of the claims 1 to 9, **characterised in that** the flip-flop circuit (1)) is a Schmitt trigger.

11. An air bubble detector comprising an ultrasonic transmitter (20) in accordance with one of the claims 1 - 10, having an ultrasonic receiver (21) and having a hose (11) attached between the ultrasonic transmitter (20) and the ultrasonic receiver (21).

## Revendications

1. Émetteur d'ultrasons (20), notamment pour un détecteur de bulles d'air, doté d'un palier d'émission (23) et d'un générateur d'ultrasons (5),
**caractérisé en ce que**
le palier d'émission (23) présente un multivibrateur (22), qui est composé d'une bascule électronique (1) présentant deux états de sortie stables et d'un élément temporel (2, 3) commuté dans la branche de rétroaction de la bascule électronique, et
**en ce que** le générateur d'ultrasons (5) est commuté avec la branche de rétroaction de la bascule électronique de manière à ce que le palier d'émission (23) oscille sur à proximité d'une fréquence de résonance du générateur d'ultrasons.

2. Émetteur d'ultrasons selon la revendication 1, **caractérisé en ce que** l'élément temporel du multivibrateur est composé d'au moins un élément RC (2, 3).

3. Émetteur d'ultrasons selon la revendication 2, **caractérisé en ce que** le générateur d'ultrasons (5) est commuté en parallèle à la résistance (2) de l'élément RC.

4. Émetteur d'ultrasons selon une quelconque des revendications 1 à 3, **caractérisé en ce que**, sur la sortie de la bascule électronique, un passe-bas (6, 7) est prévu afin d'inhiber un amorçage des oscillations du générateur d'ultrasons (5) sur les oscillations supérieures de celui-ci.

5. Émetteur d'ultrasons selon la revendication 4, **caractérisé en ce que** le générateur d'ultrasons (5) est exploité à sa fréquence de résonance en série inférieure.

6. Émetteur d'ultrasons selon une quelconque des revendications 1 à 5, **caractérisé en ce que** le palier d'émission oscille au-dessous d'une fréquence de résonance correspondante du générateur d'ultrasons.

7. Émetteur d'ultrasons selon une quelconque des revendications 1 à 6, **caractérisé en ce que** le palier d'émission (23) est commuté logiquement avec une entrée d'essai (9), afin d'activer ou de désactiver l'émetteur d'ultrasons de manière ciblée à des fins d'essais.

8. Émetteur d'ultrasons selon la revendication 7, **caractérisé en ce que** l'entrée d'essai (9) est commutée par une porte ET (10) avec l'entrée de la bascule électronique (1).

9. Émetteur d'ultrasons selon une quelconque des revendications 1 à 8, **caractérisé en ce que** le générateur d'ultrasons (5) est un élément piézo.

10. Émetteur d'ultrasons selon une quelconque des revendications 1 à 9, **caractérisé en ce que** la bascule électronique (1) est un déclencheur de Schmitt.

11. Détecteur de bulles d'air avec un émetteur d'ultrasons (20) selon une quelconque des revendications 1-10, avec un récepteur d'ultrasons (21) et un tuyau (11) intégré entre l'émetteur d'ultrasons (20) et le récepteur d'ultrasons (21).
